# EUROPÄISCHE PATENTSCHRIFT

(11) **EP 1 033 108 B1**
(45) Veröffentlichungstag und Bekanntmachung des Hinweises auf die Patenterteilung: **29.03.2006**
(21) Anmeldenummer: 00810084.4
(22) Anmeldetag: 31.01.2000
(51) Int. Cl.: A61B 17/16, A61F 2/36

(54) **Knochenraspel für eine Femurkopfprothese**
Medullary broach for a femoral prosthesis
Râpe à os pour une prothèse fémorale

(30) Priorität: 04.03.1999 EP 99810186
(43) Veröffentlichungstag der Anmeldung: 06.09.2000
(73) Patentinhaber: Zimmer GmbH, 8404 Winterthur (CH)
(72) Erfinder: Spotorno, Lorenzo, 17024 Finale Ligure (IT); Lechner, Markus, 8134 Adliswil (CH)
(74) Vertreter: Manitz, Finsterwald & Partner GbR

(56) Entgegenhaltungen:
- EP-A- 0 378 044
- EP-A- 0 563 585
- US-A- 4 921 493

## Beschreibung

Die Erfindung betrifft eine Knochenraspel für eine Femurkopfprothese gemäss Oberbegriff des Anspruches 1. Eine solche Knochenraspel ist aus EP 0 563 585 A1 bekannt.

Die bekannten Raspeln haben einen länglichen, konischen Körper mit einem rechteckigen Querschnitt. Am Körper sind Schneidrippen ausgebildet, welche quer zur Längsachse des Körpers angeordnet sind und welche die gleiche Querschnittform und Abmessung aufweisen.

Bei der Anwendung der Raspel wird im Wesentlichen die gleiche Menge über die Länge abgetragen. Als nachteilig erweist sich, dass hierbei insbesondere in Bereichen mit grösserem Konuswinkel eine übermässige Menge abgetragen wird oder ein Blockieren der Raspel stattfindet, obwohl sie sich selbst durch allseitig angreifende Kräfte noch nicht zentriert hat. Für einen Operateur ist es daher schwierig, den Widerstand der Raspel beim Einschlagen richtig zu interpretieren.

Die Erfindung wie sie in den Ansprüchen gekennzeichnet ist, löst die Aufgabe eine Knochenraspel für eine Femurprothese zu verbessern.

Eine Eigenschaft liegt darin, dass bei gegebener Schneidengeometrie der Spanraum bei kleinerem Schneidenabstand überproportional abnimmt. Die Spanräume zwischen den Schneiden mit kleinem Abstand, welche an Orten mit einem grösseren Konuswinkel angebracht sind, füllen sich relativ schnell und verhindern einen zu grossen lokalen Abtrag. Beim Einschlagen der Raspel gleichen sich die radial an der Raspel auftretenden Kräfte derart aus, dass eine Selbstführung in der Markraumhöhle entsteht, bei der die relativ dünnwandige Kortikalis medial im proximalen Teil erhalten bleibt.

Im Gegensatz zu Hohlraspeln und Raspeln mit einem übergrossen Spanraum werden die Knochenspäne wegen der gezielt angepassten Hinterschneidungen nicht aus der Markraumhöhle ausgetragen, sondern mit jedem neuen Einschlagen tiefer gestossen und in den nicht an der Raspel anliegenden Bereichen verdichtet, bis die Raspel vollumfänglich Kontakt zu angeschnittenem oder zu verdichtetem Knochenmaterial hat.

Nachfolgend wird die Erfindung anhand der beiliegenden Figuren erläutert.

Es zeigen:
- Fig. 1: Eine Ansicht einer Ausführungsform einer erfindungsgemässen Knochenraspel;
- Fig. 2: eine Einzelheit A in Fig. 1;
- Fig. 3: eine Einzelheit B in Fig. 1 und
- Fig. 4: eine Ansicht einer weiteren Ausführungsform einer in einem Femurknochen eingesetzten Knochenraspel.

Die hier in Rede stehende Knochenraspel entspricht in der Form praktisch einer Femurkopfprothese, so dass eine Prothese in die vorbereitete Höhle direkt eingesetzt oder einzementiert werden kann.

Bei direkt eingesetzten Prothesen kann die zugehörige Knochenraspel im Bereich von Längsrippen der Prothese ein Untermass aufweisen, das beispielsweise der halben Rippenhöhe der Prothese entspricht. Ein Untermass von 20 % bis 70 % ist je nach Konfiguration der Längsrippen denkbar.

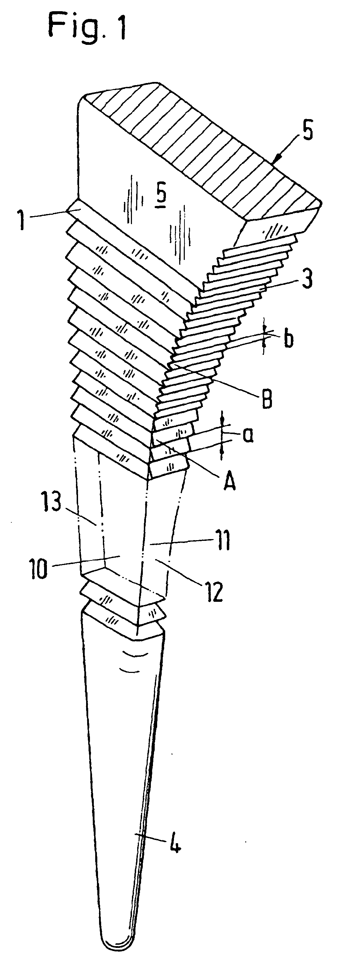

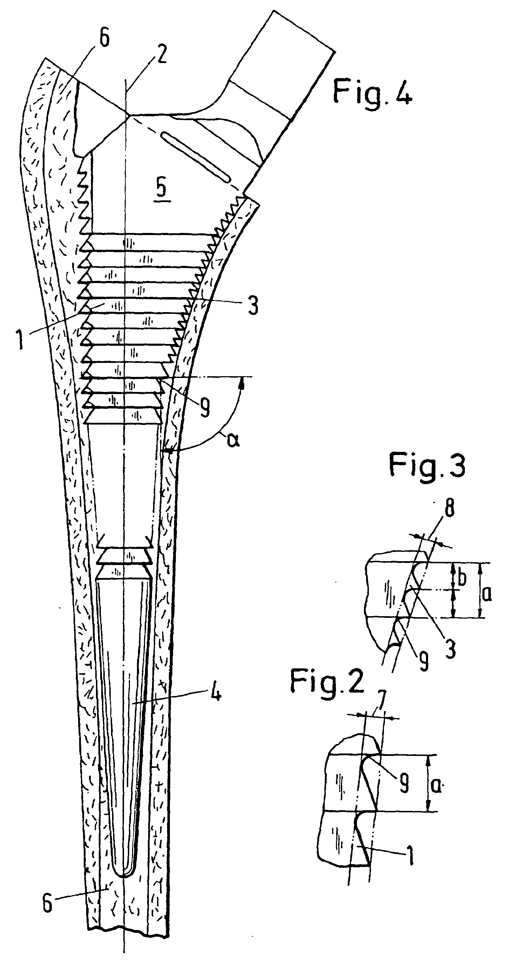

Die Fig. 1 zeigt eine Raspel mit einem länglichen Körper, dessen Seitenflächen 10, 11, 12, 13 im Wesentlichen einen rechteckigen Querschnitt bilden und der sich zum distalen Ende hin verjüngt, mit einer Mehrzahl von ersten Schneidrippen 1, die quer zur Längsachse 2 des Körpers und in einem ersten Abstand a parallel gestuft in der Richtung der Längsachse am Körper angeordnet sind, mit zweiten Schneidrippen 3, die in einem zweiten Abstand b parallel zueinander an mindestens einer Seitenfläche des Körpers ausgebildet sind und mit einem Abschnitt 4, der am distalen Ende ausgebildet und mit einer glatten Oberfläche versehen ist. Die ersten Schneidrippen 1 sind an der lateralen und medialen Seite sowie an der posterioren und anterioren Seite des Körpers ausgebildet. Die zweiten Schneidrippen 3 sind im Bereich mit dem grösseren Konuswinkel an der medialen Seite des Körpers ausgebildet. Wie in den Figuren 2 und 3 ersichtlich, sind die Abstände der zweiten Schneidrippen 3 kleiner als die Abstände der ersten Schneidrippen 1. Mit Vorteil wird für die zweiten Schneidrippen 3 ein Abstand b gewählt, der einem Bruchteil des Abstandes a der ersten Schneidrippen 1 mit einem ganzzahligen Kehrwert entspricht, z.B. 1/2, 1/3. Somit weisen die ersten und zweiten Schneidrippen 1 und 3 bei ähnlicher Schneidengeometrie eine unterschiedliche Spanungstiefe 7, 8 auf, die zusätzlich durch die Wahl des Freiwinkels veränderbar ist, so dass unterschiedliche Mengen abgetragen werden. Im proximalen Bereich sind auf der posterioren und der anterioren Seite jeweils Abschnitte 5 mit glatter Oberfläche vorgesehen.

Zum besseren Verständnis der Erfindung ist in Fig. 4 ein Femurknochen dargestellt, in welchen eine derartige Raspel eingesetzt ist. Beim Einsetzen der Raspel in den Hohlraum im Knochen 6 erfolgt eine Vororientierung durch den Abschnitt 4 am distalen Ende. Beim Einschlagen der Raspel wird durch die Schneidrillen 1 und 3 Material abgetragen, wobei im Bereich des grösseren Konuswinkels an der medialen Seite weniger Material abgetragen wird. Hier wird die Raspel an der medialen Seite geführt, wodurch das Einschlagen vereinfacht wird. Das durch die Schneidrillen 1 und 3 abgetragene Material wird mittels dem Abschnitt 4 am distalen Ende der Raspel bzw. den Abschnitten 5 am proximalen Ende der Raspel verdichtet. Zur Kontrolle der Stellung der einzusetzenden Prothese bezüglich der Hüftschale kann eine Kontrollkugel (nicht dargestellt) auf die Raspel aufgesetzt werden. Nachdem die Raspel entfernt ist, kann eine Femurkopfprothese in den vorbereiteten Hohlraum direkt eingesetzt oder einzementiert werden.

Die Führung beim Schneiden und die Freigabe von zerspantem Material beim Herausschlagen der Raspel kann durch die Veränderung des Schneidwinkels α der Schnittflächen 9 zur Längsachse 2 der Raspel und durch eine Veränderung des Freiwinkels der Schneiden 1, 3 zusätzlich verändert werden. So wird beispielsweise mit einem Schneidwinkel α > 90° weniger geschnitten und mehr verdichtet, während mit einer Verkleinerung des Freiwinkels durch eine Verkleinerung des Spanraumes die geschnittene Menge begrenzt und beim Rückziehen leichter abgegeben wird.

Auf diese Weise ist es möglich, anschliessend an den distalen glatten Abschnitt 4 eine Zone mit allseitig zweiten Schneidrippen 3 angrenzen zu lassen, die mehr verdichten als schneiden.

## Patentansprüche

1. Knochenraspel für eine Femurkopfprothese mit einem länglichen sich gegen distal verjüngenden Körper, dessen Seitenflächen (10,11, 12, 13) einen im Wesentlichen rechteckigen Querschnitt bilden, mit einer Mehrzahl von ersten Schneidrippen (1), die quer zur Längsachse (2) des Körpers verlaufen und in einem ersten Abstand a parallel gestuft längs der Längsachse angeordnet sind, und mit zweiten Schneidrippen (3) die in einem zweiten Abstand b parallel gestuft an mindestens einer Seitenfläche des Körpers ausgebildet sind, wobei die ersten und zweiten Schneidrippen (1, 3) eine unterschiedlich grosse Spanungstiefe (7, 8) aufweisen, um unterschiedliche Mengen abzutragen,
**dadurch gekennzeichnet, dass** im proximalen Bereich auf der posterioren und anterioren Seite (10, 11) jeweils ein Abschnitt (5) mit glatter Oberfläche vorgesehen ist der sich zwischen Anspruch 9 und Anspruch 10 befindenden Text einfügen.

2. Raspel nach Anspruch 1, **dadurch gekennzeichnet, dass** die ersten und zweiten Schneidrippen (1 und 3) gruppenweise angeordnet sind.

3. Raspel nach Anspruch 1, **dadurch gekennzeichnet, dass** die zweiten Schneidrippen (3) an der medialen Seite des Körpers im Bereich des grösseren Konuswinkels angeordnet sind.

4. Raspel nach einem der Ansprüche 1 bis 3, **dadurch gekennzeichnet, dass** der zweite Abstand b kleiner als der erste Abstand a ist.

5. Raspel nach einem der Ansprüche 1 bis 4, **dadurch gekennzeichnet, dass** die zweiten Schneidrippen (3) eine geringere Spanungstiefe (8) als die Spanungstiefe (7) der ersten Schneidrippen (1) aufweisen.

6. Raspel nach einem der Ansprüche 1 bis 5, **dadurch gekennzeichnet, dass** Schnittflächen (9) der Schneidrippen (1, 3) mit der Längsachse (2) einen Schneidwinkel α grösser 90° bilden.

7. Raspel nach Anspruch 4, **dadurch gekennzeichnet, dass** der zweite Abstand b ein Bruchteil des ersten Abstandes a ist, dessen Kehrwert einer ganzen Zahl entspricht.

8. Raspel nach einem der Ansprüche 1 bis 7, **dadurch gekennzeichnet, dass** die Schneidrippen (1, 3) von einer Seitenfläche (10, 11, 12, 13) zu einer benachbarten Seitenfläche in der Richtung der Längsachse (2) zueinander versetzt sind.

9. Raspel nach einem der Ansprüche 1 bis 8, **gekennzeichnet durch** einen Abschnitt (4) am distalen Ende, der mit einer glatten Oberfläche versehen ist.

10. Raspel nach Anspruch 9, **dadurch gekennzeichnet, dass** oberhalb des glatten distalen Abschnitts (4) eine Zone mit allseitig zweiten Schneidrippen (3) angrenzt.

11. Prothesensystem bestehend aus einer Raspel nach einem der Ansprüche 1 bis 10 und einer Femurkopfprothese mit Längsrippen, die vorgesehen ist für ein Einsetzen nach dem Raspeln eines Knochenbetts, **dadurch gekennzeichnet, dass** die Raspel in Bereichen, in denen die Prothese Längsrippen aufweist mit einem Untermass von 20 % bis 70 % der Rippenhöhe der Prothese versehen ist.

## Claims

1. Bone rasp for a femur head prosthesis having an elongate body which tapers toward distal, the side surfaces (10, 11, 12, 13) of which form a substantially rectangular cross-section, having a plurality of first cutting ribs (1) which extend transversely to the longitudinal axis (2) of the body and are arranged in parallel graduations at a first spacing a along the longitudinal axis and having second cutting ribs (3) which are formed in parallel graduations at a second spacing b at at least one side surface of the body, with the first and second cutting ribs (1, 3) having a different depth of cut (7, 8) in order to remove different amounts, **characterised in that** a respective section (5) with a smooth surface is provided at each of the posterior and anterior sides (10, 11) in the proximal region.

2. Rasp in accordance with claim 1, **characterised in that** the first and second cutting ribs (1 and 3) are arranged group-wise.

3. Rasp in accordance with claim 1, **characterised in that** the second cutting ribs (3) are arranged at the medial side of the body in the region of the greater cone angle.

4. Rasp in accordance with any one of the claims 1 to 3, **characterised in that** the second spacing b is smaller than the first spacing a.

5. Rasp in accordance with any one of the claims 1 to 4, **characterised in that** the second cutting ribs (3) have a smaller depth of cut (8) than the depth of cut (7) of the first cutting ribs (1).

6. Rasp in accordance with any one of the claims 1 to 5 **characterised in that** cutting surfaces (9) of the cutting ribs (1, 3) form a cutting angle α greater than 90° with the longitudinal axis (2).

7. Rasp in accordance with claim 4, **characterised in that** the second spacing b is a fraction of the first spacing a, the reciprocal value of which corresponds to a whole number.

8. Rasp in accordance with any one of the claims 1 to 7, **characterised in that** the cutting ribs (1, 3) are displaced with respect to one another in the direction of the longitudinal axis (2) from one side surface (10, 11, 12, 13) to an adjacent side surface.

9. Rasp in accordance with any one of the claims 1 to 8, **characterised by** a section (4) at the distal end which is provided with a smooth surface.

10. Rasp in accordance with claim 9, **characterised in that** a zone with second cutting ribs (3) on all sides is adjacent above the smooth distal section (4).

11. Prosthesis system consisting of a rasp in accordance with any one of the claims 1 to 10 and a femur head prosthesis with longitudinal ribs, which is provided for an insertion after the rasping of a bone bed, **characterised in that** the rasp is provided with an under-dimensioning of 20 % to 70 % of the rib height of the prosthesis in regions in which the prosthesis has longitudinal ribs.

## Revendications

1. Râpe à os pour une prothèse de tête fémorale, comportant un corps allongé qui va en se rétrécissant en direction distale, dont les surfaces latérales (10, 11, 12, 13) forment une section transversale sensiblement rectangulaire, une pluralité de premières nervures de coupe (1) qui s'étendent transversalement à l'axe longitudinal (2) du corps et qui sont agencées parallèlement à une première distance a échelonnées le long de l'axe longitudinal, et comportant des secondes nervures (3) qui sont réalisées parallèlement à une seconde distance b échelonnées sur au moins une surface latérale du corps, les premières et les secondes nervures de coupe (1, 3) présentant des profondeurs de coupe (7, 8) de différentes tailles, afin d'enlever différentes quantités, **caractérisée en ce qu'**il est prévu dans la zone proximale, sur les côtés postérieur et antérieur (10, 11), une portion respective (5) présentant une surface lisse.

2. Râpe selon la revendication 1, **caractérisée en ce que** les premières et les secondes nervures de coupe (1 et 3) sont agencées par groupes.

3. Râpe selon la revendication 1, **caractérisée en ce que** les secondes nervures de coupe (3) sont agencées sur le côté médial du corps dans la zone du plus grand l'angle de cône.

4. Râpe selon l'une des revendications 1 à 3, **caractérisée en ce que** la seconde distance b est inférieure à la première distance a.

5. Râpe selon l'une des revendications 1 à 4, **caractérisée en ce que** les secondes nervures de coupe (3) présentent une profondeur de coupe (8) inférieure à la profondeur de coupe (7) des premières nervures de coupe (1).

6. Râpe selon l'une des revendications 1 à 5, **caractérisée en ce que** les surfaces de coupe (9) des nervures de coupe (1, 3) forment avec l'axe longitudinal (2) un angle de coupe α supérieur à 90°.

7. Râpe selon la revendication 4, **caractérisée en ce que** la seconde distance b est une fraction de la première distance a dont la valeur inverse correspond à un nombre entier.

8. Râpe selon l'une des revendications 1 à 7, **caractérisée en ce que** d'une surface latérale (10, 11, 12, 13) à une surface latérale voisine, les nervures de coupe (1, 3) sont décalées les unes par rapport aux autres dans la direction de l'axe longitudinal (2).

9. Râpe selon l'une des revendications 1 à 8, **caractérisée par** une portion (4) à l'extrémité distale, qui est pourvue d'une surface lisse.

10. Râpe selon la revendication 9, **caractérisée en ce qu'**une zone présentant sur tous les côtés des secondes nervures de coupe (3) est adjacente au-dessus de la portion lisse distale (4).

11. Système de prothèse constitué par une râpe selon l'une des revendications 1 à 10 et par une prothèse de tête fémorale, pourvue de nervures longitudinales, qui est prévue pour la mise en place après un râpage d'un lit osseux, **caractérisé en ce que** dans des zones dans lesquelles la prothèse comprend des nervures longitudinales, la râpe présente une dimension inférieure de 20 % à 70 % à la hauteur de nervure de la prothèse.
